# EUROPEAN PATENT APPLICATION

(11) **EP 1 997 515 A1**
(43) Date of publication of application: **03.12.2008**
(21) Application number: 07010701.6
(22) Date of filing: 30.05.2007
(51) Int. Cl.: A61K 51/04, A61K 101/00, A61K 101/02

(54) **Use of radiolabelled phenyloxyaniline derivatives for imaging diseases associated with peripheral benzodiazepin receptor's activation**

(71) Applicant: Bayer Schering Pharma Aktiengesellschaft, 13342 Berlin (DE)
(72) Inventor: Lippoldt, Andrea, Dr., 13125 Berlin (DE); Bacher-Stier, Claudia, Dr., 13156 Berlin (DE)

(57) **Abstract**

The present invention relates to the use of a specific compound, as described in detail below, having a high affinity to the peripheral benzodiazepine receptor for imaging cardiac and cardiovascular diseases for which inflammation and/or mitochondrial dysfunction and any other pathophysiological process leading to PBR activation are major contributing factor.

## Description

### FIELD OF THE INVENTION

The present invention relates to the use of a specific compound, as described in detail below, having a high affinity to the peripheral benzodiazepine receptor for imaging cardiac and cardiovascular diseases for which inflammation and/or mitochondrial dysfunction and any other pathophysiological process leading to PBR activation are major contributing factor.

### BACKGROUND OF THE INVENTION

It's estimated for the year 2004 that 79,400,000 Americans had one or more forms of cardiovascular disease (CVD) having two main components:
■ Diseases of the heart (cardio)
■ Diseases of the blood vessels (vascular)

The heart diseases to be considered are coronary artery disease, ischemic or other cardiomyopathy, inflammatory valvular heart disease, inflammatory pericardial disease" heart failure of different origin, myocarditis.

A number of studies have shown that inflammation of blood vessels is one of the major factors that increase the incidence of heart diseases, including atherosclerosis (clogging of the arteries), stroke and myocardial infarction or heart attack.

The blood vessel disorders to be considered are arteriosclerosis and atherosclerosis of any vessel, high blood pressure, stroke, aneurysm, peripheral arterial disease, vasculitis, venous incompetence, venous thrombosis, lymphedema.

Kidney diseases to be considered are ischemia-reperfusion injury, acute and/or chronic renal failure, nephrosclerosis, autoimmune diseases affecting the kidneys.

Autoimmune diseases to fall under the patent e.g. rheumatoid arthritis, MS and others.

In the cardiovascular system, PBRs are expressed in blood cells, e.g. platelets, lymphocytes, and mononuclear cells. In addition, this receptor is part of the mitochondrial transition pore and known to be expressed in heart cells as well, i.e. striated cardiac muscle cells and vascular smooth muscle cells, mast cells as well as endothelial cells. Inflammation and/or mitochondrial dysfunction are involved in most of the cardiovascular diseases as well as in many other cardiac, vascular and kidney diseases. Therefore, as a consequence of any injury (trauma, ischemia) immune cells bearing the peripheral benzodiazepine receptor infiltrate into the respective organs,i.e. heart, kidneys, vascular wall, and upregulate the PBR receptor upon activation. In addition, PBRs in heart cells and blood vessel walls are altered in events leading to mitochondrial dysfunction, like in ischemia-reperfusion injury.

In the kidney, PBRs are expressed in epithelial cells of the proximal, distal tubular system including the limbs of Henle. The PBR expression is regulated upon acute renal failure due to hypoxia or ischemic events.

Benzodiazepine (BZ) receptors are classified into central and peripheral benzodiazepine receptors. A peripheral benzodiazepine receptor (PBR) was at first confirmed in the periphery but its presence in the central nervous system was noted as well. It has been further clarified that PBR has a high density in the central nervous system and the density is same as or even higher than that of a central benzodiazepine receptor (CBR) in the same region. According to recent studies, it has been reported that PBR is present in microglia cells in the brain and increases in neurodegenerative diseases such as Alzheimer's disease and auto-immune diseases, such as Multiple sclerosis, where microglia as well as macrophages are activated in the brain.

Leducq et al (JPET, vol 306, N°3, 828-837, 2003) discloses that peripheral benzodiazepine receptors play a major role in the regulation of cardiac ischemia-reperfusion injury. SSR180575 a pyridazazino-indol derivative is seen as a novel peripheral benzodiazepine receptor ligand.

It' has be found that phenyloxyaniline derivatives having a high affinity for peripheral benzodiazepine receptor can be used for imaging cardiovascular diseases for which inflammation and/or mitochondrial dysfunction are/is a major contributing factor

### SUMMARY OF THE INVENTION

The object of the present invention is to provide the use of the compound which is useful as a ligand for PBR, having a strong affinity and a high selectivity and, in an external measurement of PBR where a sufficient signal has not been obtained until now, to label a ligand of PBR having a high affinity and a high selectivity with a positron nuclide whereby measurement of PBR in a living body is made possible for imaging cardiovascular diseases and more particularly vessel, heart and kidney diseases for which inflammation and/or mitochondrial dysfunction are major contributing factor. Such diseases are e.g. aneurysms, athersclerosis, cardiomyopathy and some other heart diseases, heart failure, inflammatory valvular disease, coronary artery disease, endocarditis, atheroma, arteriosclerosis, inflammatory diseases of the heart, e.g. myocarditis, inflammatory cardiomyopathy, renal diseases, autoimmune diseases and nephrosclerosis.

### DETAILED DESCRIPTION OF THE INVENTION

The invention concerns the use of compound of formula 1 for imaging vessel, heart, and kidney diseases for which inflammation and/or mitochondrial dysfunction are major contributing factor. 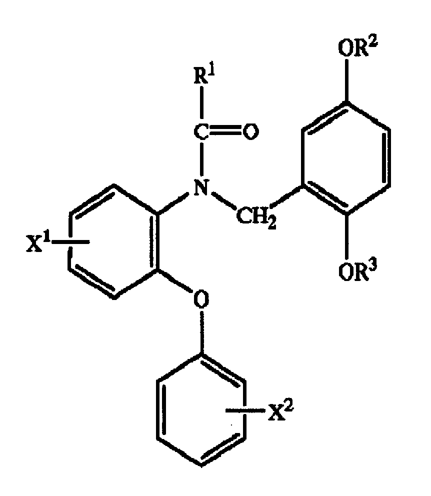 wherein X1 and X2 are independently from each other a hydrogen atom or a halogen atom, R1 and R2 are independently from each other a hydrogen atom, alkyl group having 1 to 10 carbon(s), a halogen-substituted alkyl group having 1 to 10 carbon(s) or radioisotope, and R3 is a halogen substituted alkyl group having 1 to 5 carbon(s) or a radioisotope thereof.

In a preferred embodiment of the compound of formula 1, the halogen atom in R3 is a fluorine atom, an iodine atom or a bromine atom and, more preferably, a fluorine atom or an iodine atom.

In a preferred embodiment of the compound of formula 1, the radioisotope in the present invention is ¹¹C, ¹⁸F, ¹²³I and more preferably ¹⁸ F.

In a preferred embodiment of the use the diseases are selected from aneurysms, athersclerosis, cardiomyopathy and some other heart diseases, heart failure, inflammatory valvular disease, coronary artery disease, endocarditis, atheroma, arteriosclerosis, inflammatory diseases of the heart, such as myocarditis, inflammatory cardiomyopathy, renal diseases, autoimmune diseases and nephrosclerosis.

In a preferred embodiment of the use the Kidney diseases are selected from ischemia-reperfusion injury, acute and/or chronic renal failure, nephrosclerosis, autoimmune diseases affecting the kidneys.

In a more preferred embodiment the diseases are selected from myocarditis, atheroma, arteriosclerosis.

In a preferred embodiment of the use the compound 1 is N-(2-[¹⁸F]fluoromethyl-5-methoxybenzyl-N-(5-fluoro-2-phenoxyphenyl)acetamide (hereinafter, referred to as [¹⁸F]FMDAA1106)
or
N-[2-(2-[¹⁸ F]fluoro)ethyl-5-methoxybenzyl]-N-(5-fluoro-2-phenoxyphenyl)acetamide (hereinafter, referred to as [¹⁸ F]FEDAA1106).

The alkyl group having 1 to 10 carbon(s) means a linear or branched alkyl group and its examples are a methyl group, an ethyl group, an n-propyl group, an isopropyl group, an n-butyl group and an n-heptyl group.
The halogen-substituted alkyl group having 1 to 10 carbon(s) means a linear or branched alkyl group where 1 to 3 halogen atom(s) is/are substituted for a hydrogen atom(s) and, preferably, it is an alkyl group substituted with fluorine or iodine atom(s). Examples thereof are a fluoromethyl group, a 2-fluoroethyl group, an 2-iodoethyl group, a 5-fluoroheptyl group and a 6-bromohexyl group. The halogen-substituted alkyl group having 1 to 5 carbon(s) means a linear or branched alkyl group where 1 to 3 halogen atom(s) is/are substituted for a hydrogen atom(s) and, preferably, it is an alkyl group substituted with fluorine or iodine atom(s). Examples thereof area fluoromethyl group, a 2-fluoroethyl group, an 2-iodoethyl group and a 5-fluoroheptyl group.

In a second aspect of the invention, the invention relates to the use of compound of formula 1 for imaging peripheral inflammatory disease, characterized by infiltration of white blood cells, e.g. osteomyelitis or rheumatoid arthritis for which inflammation and/or mitochondrial dysfunction are/is a major factor of incidence.

In a third aspect of the invention, the invention relates to the use of compound of formula 1 for imaging autoimmune diseases. In a more preferred embodiment the autoimmune disease is selected from Multiple sclerosis, rheumatoid arthritis, Amyotrophic lateral sclerosis (ALS), and others

### EXAMPLES

The present invention will now be illustrated in more detail by way of the following Examples.

### Example 1

Production of N-(2-fluoromethyl-5-methoxybenzyl)-N-(5-fluoro-2-phenoxyphenyl)acetamide (hereinafter, referred to as FMDAA1106) An oily sodium hydride (60%) (5.1 mg) was added to a solution of N-(2-hydroxy-5-methoxybenzyl)-N-(5-fluoro-2-phenoxyphenyl)acetamide (hereinafter, referred to as DAA1123) (18 mg) in N,N-dimethylformamide (DMF; 1.0 mL) followed by stirring at 0[deg.] C., 10 mg of fluoromethyl iodide (FCH2I) were added thereto and the mixture was further stirred at 0[deg.] C. for 1 hour. Water was added to the reaction solution, the mixture was extracted with ethyl acetate and the organic layer was washed with saturated brine and dried over anhydrous magnesium sulfate. The solvent was evaporated under reduced pressure and the resulting crude product was purified by a silica gel column chromatography (chloroform:hexane:ethyl acetate=1:3:1) to give 16 mg of the above-identified compound.
Melting point: 71 to 72°C.

### Example 2

Production of N-(2-[¹⁸ F]fluoromethyl-5-methoxybenzyl-N-(5-fluoro-2-phenoxyphenyl)acetamide (hereinafter, referred to as [¹⁸F]FMDAA1106) [¹⁸ F]fluorine ([¹⁸ F]F) was produced by irradiation of 18 MeV proton using 20 atom.% H218 O. After the irradiation, [¹⁸ F]F was recovered from a target, separated from [¹⁸O]H2O by an anion-exchange resin Dowex 1-X8, mixed with acetonitrile (CH3CN, 1.5 mL) containing Kryptofix 2.2.2. (25 mg) and transferred from an irradiation chamber to a synthetic cell. After [¹⁸ F]F was dried in a synthetic cell, diiodomethane (CH2I2) was injected into a reactor at 130[deg.] C. Together with the injection, [¹⁸ F]F CH2I produced by helium gas was bubbled into a solution of 1 mg of DAA1123 and sodium hydride (6.8 [mu]L, 0.5 g/20 mL) in DMF (300 mL). After this process was maintained at room temperature for 10 minutes, the reaction mixture was injected into a reversed phase semi-separation HPLC (YMCJ' sphere ODS-H80 column; 10 mm ID*250 mm). A fraction of [¹⁸ F]FMDAA1106 was collected where the mobile phase was CH3CN/H2O at the flow rate of 6 mL/minute. The solvent was evaporated under reduced pressure from this fraction and the residue was dissolved in saline (10 mL) and passed through a 0.22-[mu]m Millipore filter to give [<18> F]FMDAA1106 (110 MBq, n=3) as the final preparation. (Condition for irradiation; 15 minutes, 15 [mu]A). Incidentally, the synthetic time needed was about 45 minutes from the completion of the irradiation.

### Example 3

Production of N-[2-(2-fluoro)ethyl-5-methoxybenzyl]-N-(5-fluoro-2-phenoxyphenyl)acetamide (hereinafter, referred to as FEDAA1106) The same operation as in Example 1 was carried out where 1-fluoro-2-tosyloxyethane (FCH2CH2OTs) was used instead of fluoromethyl iodide (FCH2I) to give 20 mg of the above-identified compound. Melting point: 54 to 56° C.

### Example 4

Production of N-[2-(2-[¹⁸F]fluoro)ethyl-5-methoxybenzy]-N-(5-fluoro-2-phenoxyphenyl)acetamide (hereinafter, referred to as [¹⁸F]FEDAA1106) The same operation as in Example 2 was carried out where 2-bromoethyl triflate (BrCH2CH2OTf) was used instead of diiodomethane (CH2I2) to give the above-identified compound.

In accordance with the present invention, there is provided a compound which is useful as a ligand for PBR having a strong affinity and a high selectivity. In an external measurement of PBR where a sufficient signal has not been obtained until now, a ligand of PBR having a high affinity and a high selectivity is labeled with a positron nuclide whereby measurement of PBR in a living body is now possible.

While the invention has been described in detail and with reference to specific examples thereof, it will be apparent to one skilled in the art that various changes and modifications can be made therein without departing from the spirit and scope thereof.

## Claims

1. Use of compound of formula 1 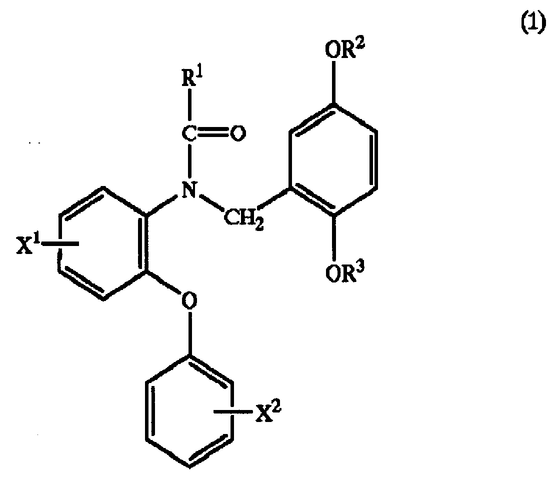 wherein X1 and X2 are independently from each other a hydrogen atom or a halogen atom, R1 and R2 are independently from each other a hydrogen atom, alkyl group having 1 to 10 carbon(s), a halogen-substituted alkyl group having 1 to 10 carbon(s) or radioisotope, R3 is a halogen substituted alkyl group having 1 to 5 carbon(s) or a radioisotope thereof
for imaging vessel, heart, and kidney diseases for which inflammation and/or mitochondrial dysfunction are major contributing factor.

2. Use according to claim 1 wherein radioisotope is ¹¹C, ¹⁸F, or ¹²³I.

3. Use according to claim 2 wherein radioisotope is ¹⁸F.

4. Use according to claim 1 wherein compound of formula 1 is N-(2-[¹⁸F]fluoromethyl-5-methoxybenzyl-N-(5-fluoro-2-phenoxyphenyl)acetamide or N-[2-(2-[¹⁸ F]fluoro)ethyl-5-methoxybenzyl]-N-(5-fluoro-2-phenoxyphenyl)acetamide.

5. Use according to claims 1 to 4 wherein imaging vessel, heart and kidney diseases for which inflammation and/or mitochondrial dysfunction are/is a major factor of incidence are diseases selected from aneurysms, angina, arrhythmia, athersclerosis, cardiomyopathy, congenital heart disease, congestive heart failure, myocarditis, valve disease, coronary artery disease, dilatated cardiomyopathy, diastolic dysfunction, endocarditis, atheroma, arteriosclerosis, inflammatory diseases of the heart, like myocarditis, inflammatory cardiomyopathy, acute renal failure, nephrosclerosis.

6. A method for detecting vessel, heart and kidney diseases for which inflammation and/or mitochondrial dysfunction are major contributing factor using a compound of formula 1 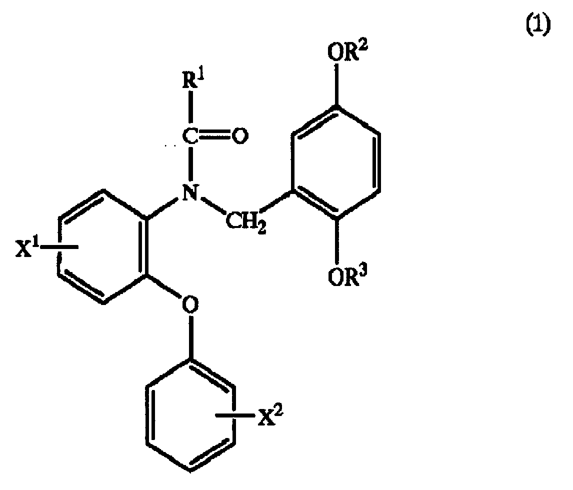 Wherein X1 and X2 are independently from each other a hydrogen atom or a halogen atom, R1 and R2 are independently from each other a hydrogen atom, alkyl group having 1 to 10 carbon(s), a halogen-substituted alkyl group having 1 to 10 carbon(s) or radioisotope, R3 is a halogen substituted alkyl group having 1 to 5 carbon(s) or a radioisotope thereof
comprising the step of injecting to a patient a radiolabled compound of formula 1 and then scanning patient with a PET scanner.

7. Use of compound of formula 1 for imaging peripheral inflammatory disease.

8. Use of compound of formula 1 for the imaging of autoimmune diseases.

9. Use according to claim 8 wherein the autoimmune diseases is selected from Multiple sclerosis, rheumatoid arthritis or Amyotrophic lateral sclerosis (ALS).
